# EUROPÄISCHE PATENTANMELDUNG

(11) **EP 2 940 613 A1**
(43) Veröffentlichungstag der Anmeldung: **04.11.2015**
(21) Anmeldenummer: 15164077.8
(22) Anmeldetag: 17.04.2015
(51) Int. Cl.: G06F 19/00

(54) **DATENVERARBEITUNGS- UND KOMMUNIKATIONSEINRICHTUNG ZUR AUFNAHME VON PATIENTENDATEN IN THERAPIEFREIER ZEIT**

(30) Priorität: 28.04.2014 DE 102014105916
(71) Anmelder: B. Braun Avitum AG, 34212 Melsungen (DE)
(72) Erfinder: Henze, Janosch, 34121 Kassel (DE); Strohhöfer, Christof, Dr., 34123 Kassel (DE)
(74) Vertreter: Winter, Brandl, Fürniss, Hübner, Röss, Kaiser, Polte - Partnerschaft mbB

(57) **Zusammenfassung**

Offenbart ist ein Patientendatenüberwachungssystem zum zentralen Überwachen von zumindest einem Vitalparameter zumindest eines Dialysepatienten in der therapiefreien Zeit, das aufweist: eine zentrale Patientendatenbank (6) zum Abspeichern von Patientendaten, welche über eine Kommunikationsschnittstelle von Ärzten, Klinikpersonal und/oder dem entsprechenden Patienten jederzeit einsehbar sind; und zumindest ein, von einem Patienten in der therapiefreien Zeit mitgeführtes oder zu Hause vorgehaltenes, Messgerät (3) zum Messen zumindest eines Vitalparameters des zumindest einen Patienten. Erfindungsgemäß ist eine von dem zumindest einen Patienten in der dialysefreien Zeit mitgeführte oder zu Hause vorgehaltene, insbesondere transportable, Datenverarbeitungs- und Kommunikationseinrichtung (1) vorgesehen, welche jedem gemessenen und über eine Datenverbindung vom dem zumindest einen Messgerät (3) erhaltenen Vitalparameter eine entsprechende Patienten-ID zuweist, selbsttätig eine Ferndatenverbindung zu der zentralen Patientendatenbank (6) aufbaut, und selbsttätig, sobald die Verbindung zur zentralen Patientendatenbank (6) aufgebaut ist, einen Datensatz aus Patienten-ID und dem zumindest einen gemessenen Vitalparameter an die zentrale Patientendatenbank (6) überträgt.

## Beschreibung

Die vorliegende Erfindung bezieht sich auf den Bereich medizinischer Geräte, insbesondere auf ein Patientendatenüberwachungssystem zur zentralen Überwachung von zumindest einem Vitalparameter zumindest eines Dialysepatienten in der therapiefreien Zeit gemäß Anspruch 1 und ein Verfahren zur zentralen Überwachung von zumindest einem Vitalparameter zumindest eines Dialysepatienten in der dialysefreien Zeit gemäß Anspruch 8.

### Hintergrund der Erfindung

Damit der Arzt einem Patienten die bestmögliche Dialysetherapie bieten kann, ist es wichtig, den Verlauf der Vitalparameter eines Patienten zu kennen. Oftmals besitzt der Arzt allerdings nur Vitalparameter zu Zeiten, in denen sich der Patient bei der Behandlung (z.B. im Dialysezentrum) befindet. Um dem Patienten eine optimale Therapie zu bieten, fehlen dem Arzt also Informationen über den Zeitraum, während der Patient zu Hause oder im Urlaub ist.

### Stand der Technik

Aktuell ist es Nephrologen nicht auf einfachem Wege möglich, Daten von Patienten, wie beispielsweise Blutdruck oder Gewicht, außerhalb der dialysepflichtigen Zeit aufzunehmen und deren Verlauf zu beobachten. Solche Informationen über die Vitalparameter der Patienten würden dazu beitragen, die Behandlung des Dialysepatienten zu verbessern. Leider stehen derzeit diese Daten entweder gar nicht oder nur unter aufwändiger Mitwirkung des Patienten zur Verfügung. Es existieren zwar Messgeräte, die Patientendaten in der dialysefreien Zeit aufnehmen können und eine Schnittstelle besitzen, die das Auslesen von Messdaten ermöglicht. Allerdings ist es nicht möglich, über diese Schnittstelle Daten direkt an eine zentrale Stelle, z.B. das Dialysezentrum, zu senden oder diese über einen längeren Zeitraum aufzunehmen und zu vergleichen.

US 2013/0211 206 A1 beschreibt den Vorgang einer Datenaufnahme während einer Dialysetherapie. Die aufgenommenen Daten werden gesammelt und aufbereitet. Die aufbereiteten Daten können dann zur Verbesserung nachfolgender Therapien genutzt werden.

US 2010/0056 878 A1 beschreibt ein mobiles Gerät, welches in der Lage ist, über Sensormessungen den Gesundheitsstatus des Patienten abzurufen. Dabei ist das mobile Gerät mit einem am Körper befestigen Sensor verbunden und nimmt von dort die Daten entgegen.

US 2009/0306 573 A1 beschreibt eine Vorrichtung zur Aufnahme von Sensordaten der Dialysemaschine und deren Weiterleitung zu einem Server (Remote-Host). Die Dialysemaschine hat eine bidirektionale, drahtlose Internetverbindung mittels WLAN zu einem Gateway. Dieses Gateway ist wiederum mit einem entfernten Server (remote host) verbunden. Über diese Verbindung werden therapiebezogene Daten von der Dialysemaschine an den entfernten Server gesendet. Ein Empfang von Daten zur Steuerung der Dialysemaschine, welche vom entfernten Server gesendet werden, ist auch möglich. Die Daten werden hier nicht während einer therapiefreien Zeit erfasst. Zudem handelt es sich nicht um eine mobile Datenerfassung.

US 2008/0294 058 A1 beschreibt ein System, um einen Patientenpuls zu messen. Das System besteht dabei aus einem Server, einem Sensor und einem mobilen Gerät, welches als Schnittstelle zwischen Sensor und Server steht. Dabei soll die Messung über den Sensor (das Pulsmessgerät) geschehen. Die Steuerung des Pulsmessgerätes wird dabei von dem mobilen Gerät übernommen, welches dann die Daten an den Server weiterleitet.

US 2012/0035534 A1 offenbart ein System, welches im Rahmen einer Dialyse Daten aufnehmen kann und die erhaltenen Daten auf drahtlosem Weg zu einem zentralen Datenspeicher überträgt. Das System ist mit einer Erkennung kodiert, die es erlaubt, den gesendeten Daten den entsprechende Patienten zuzuordnen. Anhand der Daten auf der zentralen Datenspeichereinrichtung kann eine Einstellung einer Ultrafiltrationsrate vorgenommen werden.

Für eine erfolgreiche Messung müssen die Patienten in der Regel eine ganze Reihe von Interaktionen durchführen, wie z.B. das Starten der Messung, Speichern und Weiterleiten der Daten, Bestätigen von eingegeben Informationen und/oder Zuordnung von Patientendaten zum Messwert. Die bisher verwendeten Systeme und Geräte sind daher weitestgehend dazu ausgelegt, lediglich von Einzelpersonen benutzt zu werden. Nutzt also mehr als ein Patient das System, ist eine eindeutige Zuordnung von Messdaten zum Patienten nicht auf einfachem Wege möglich. Ein denkbares Szenario ist zum Beispiel, dass sich in einem Haushalt zwei Dialysepatienten befinden, deren Vitalparameter überwacht werden sollen. Will man nicht jedem einzelnen Patienten ein Datenaufnahmegerät zur Verfügung stellen, was natürlich kostenintensiv ist, dann ist es vorteilhaft, nur ein Datenaufnahmegerät zu nutzen. In diesem Falle muss es ermöglicht werden, dass die Zuordnung der Daten zum richtigen Patienten erfolgt. Am besten erfolgt diese Zuordnung automatisch oder zumindest mit wenig Interaktion des Patienten. Bei einer fehlerhaften Zuordnung kann es im schlimmsten Fall daher passieren, dass ein gemessener Vitalparameter einem falschen Patienten zugeordnet wird und dadurch evtl. eine falsche Behandlung die Folge ist.

Weiter unterstützen einige Geräte nur firmeneigene oder für den jeweiligen Hersteller zertifizierte Messgeräte. Das bedeutet, dass auf dem Markt vorhandene Messgeräte unterschiedlicher Hersteller nicht einfach mit einem anderen System zusammenarbeiten können, weil die verwendeten Protokolle, beispielsweise zwischen Sender und Empfänger, nicht zueinander passen, oder sie eine bestimmte Schnittstelle oder Peripherie benötigen. Oftmals sind solche Systeme dazu fest installiert, sind nicht transportabel, nehmen nur Daten während einer Therapie (bei der Dialyse) auf und geben keine Behandlungsempfehlungen an den behandelnden Arzt.

Aufgabe der vorliegenden Erfindung ist es, die vorgenannten Nachteile, insbesondere die bei der Nutzung der Datenverarbeitungs- und Kommunikationseinrichtung (Datenbox) in einem Mehrpersonenhaushalt auftretenden Probleme, zu vermeiden und ein System und ein Verfahren bereitzustellen, welches eine Aufnahme von Vitalparametern mindestens eines Patienten in einer therapiefreien Zeit erlaubt ohne dass sich dieser in der Nähe des Dialysezentrums oder eines Dialysegerätes befindet, die Daten eindeutig einem Patienten zuordnet und in einer zentralen Patientendatenbank abspeichert und bereitstellt.

Diese Aufgabe wird durch ein Patientendatenüberwachungssystem mit den Merkmalen des Patentanspruchs 1 sowie durch ein Verfahren mit den Merkmalen des Anspruchs 6 gelöst. Vorteilhafte Ausgestaltungen der Erfindung sind Gegenstand der Unteransprüche.

Das Patientendatenüberwachungssystem zum zentralen Überwachen von zumindest einem Vitalparameter zumindest eines Dialysepatienten in der therapiefreien Zeit, weist eine zentrale Patientendatenbank zum Abspeichern von Patientendaten auf, welche über eine Kommunikationsschnittstelle von Ärzten, Klinikpersonal und/oder dem entsprechenden Patienten jederzeit einsehbar sind. Weiter weist es zumindest ein, von einem Patienten in der therapiefreien Zeit mitgeführtes oder zu Hause vorgehaltenes Messgerät zum Messen zumindest eines Vitalparameters des zumindest einen Patienten auf, wobei eine, von dem zumindest einen Patienten, in der dialysefreien Zeit mitgeführte oder zu Hause vorgehaltene Datenverarbeitungs- und Kommunikationseinrichtung, die jedem gemessenen und über eine Datenverbindung von dem zumindest einen Messgerät erhaltenen Vitalparameter eine entsprechende Patienten-ID, bestehend beispielsweise aus Namen des Patienten, einer eingegebenen Ziffernfolge, einem persönlichen Schlüssel oder ähnliche personalisierende Merkmale, zuordnet, selbsttätig eine Ferndatenverbindung zu der zentralen Patientendatenbank aufbaut und selbsttätig, sobald die Verbindung zur zentralen Patientendatenbank aufgebaut ist, einen Datensatz aus Patienten-ID und dem zumindest einen gemessenen Vitalparameter an die zentrale Patientendatenbank überträgt.

Die Speicherung der Daten an einer zentralen Stelle ermöglicht es dem behandelnden Arzt, den Verlauf der gespeicherten Werte über den Zeitraum der dialysefreien Zeit in die Optimierung der Behandlung mit einzubeziehen. Mit Hilfe der Datenverarbeitungs- und Kommunikationseinrichtung ist es möglich, die Patientendaten (Vitalparameter) aufzuzeichnen. Die Aufzeichnung der Daten geschieht dabei über eine Schnittstelle der Datenverarbeitungs- und Kommunikationseinrichtung, die mit einer Schnittstelle der Messgeräte kommuniziert.

Das Messgerät überträgt die gemessenen Vitalparameter in die Datenverarbeitungs- und kommunikationseinrichtung. Dabei können die Daten spätestens auf der Datenverarbeitungs- und Kommunikationseinrichtung automatisch Informationen zur Identifikation des Patienten (Patienten-ID), z.B. Patienten-Nr., Versicherungs-Nr., Name (ggf. mit Geburtsdatum) zugeordnet werden.

Somit ist die Datenverarbeitungs- und Kommunikationseinrichtung so konfiguriert, dass eine eindeutige Zuordnung von gemessenen Vitalparametern und dem entsprechenden Patienten zu einer Patienten ID für den Fall gewährleistet werden kann, dass die Datenverarbeitungs- und Kommunikationseinrichtung von mehr als nur einem einzelnen Patienten zur zentralen Überwachung von Vitalparametern genutzt wird, beispielsweise in einem Mehrpersonenhaushalt in dem die Vitalparameter von zwei Patienten überwacht werden sollen. Die Datenverarbeitungs- und Kommunikationseinrichtung ist dabei in der Lage, die aufgenommenen Daten einem bestimmten Patienten zuzuordnen.

Um eine automatische Patientenzuordnung zu vollziehen, muss sich jeder Patient, der die Datenverarbeitungs- und Kommunikationseinrichtung benutzen will, einmalig, beispielsweise über eine geeignete Dateneingabevorrichtung wie eine Tastatur, einen berührungsempfindlichen Bildschirm oder einer anderen dazu geeigneten Einrichtung, an der Datenverarbeitungs- und Kommunikationseinrichtung anmelden. Das kann beispielsweise über die Eingabe seines Namens und/oder eines Passwortes geschehen. Damit ist er dauerhaft als Benutzer für die Datenverarbeitungs- und Kommunikationseinrichtung gespeichert. Alternativ kann ein personalisierter Schlüssel für den Aufbau einer sicheren Verbindung mit dem Server auf der Datenverarbeitungs-und Kommunikationseinrichtung installiert werden. Weitere denkbare Methoden zur Identifizierung eines Patienten an der Datenverarbeitungs- und Kommunikationseinrichtung sind die Beantwortung einer Frage oder dass der Patient die getätigte Messung bestätigen muss. (Gegebenenfalls wird er aufgefordert, den Benutzer zu wechseln).

Gemäß einem Aspekt der Erfindung können die Patientendaten in der Datenverarbeitungs- und Kommunikationseinrichtung zwischengespeichert werden und zwar so lange, bis die Datenverarbeitungs- und Kommunikationseinrichtung in der Lage ist, eine Verbindung zum Internet aufzubauen und Daten an einen zentralen Server oder eine dezentrale Server- Architektur zu senden.

Ein behandelnder Arzt, eine Schwester oder der Patient selbst können die Daten abrufen. Der behandelnde Arzt oder die Schwester erfahren so z.B. über die Gewichtszunahme oder den Blutdruckverlauf des Patienten.

Die Datenverarbeitungs- und Kommunikationseinrichtung kann eine Software-Anwendung auf einem Smartphone oder einem Tablet-PC sein. Eine Datenverarbeitungs- und Kommunikationseinrichtung kann aber auch als eigenständige Hardware-Plattform ausgeführt sein.

Zum Aufbau der Internetverbindung benutzt die Datenverarbeitungs- und Kommunikationseinrichtung die ihr zu Verfügung stehenden Mittel wie beispielsweise eine Mobilfunkverbindung, eine WLAN Verbindung oder eine andere geeignete Verbindung, die eine Verbindung zur Datenübertragung über das Internet ermöglicht.

Des Weiteren kann die Datenverarbeitungs- und Kommunikationseinrichtung so konfiguriert sein, dass sie eine Kommunikation mit einer Vielzahl an unterschiedlichen Messgeräten oder Messsensoren ermöglicht. Dies ist vorteilhaft für den Fall, wenn beispielsweise Messgeräte und/oder Sensoren verschiedener Hersteller verwendet werden. Nicht alle Messgeräte und/oder Sensoren haben dieselbe Schnittstelle zu der Datenverarbeitungs- und Kommunikationseinrichtung. Beispielsweise kann ein Messgerät über eine Bluetooth-Schnittstelle mit der Datenverarbeitungs- und Kommunikationseinrichtung kommunizieren, ein anderes hat eine WLAN- oder Infrarotschnittstelle. Dazu kann die Datenverarbeitungs- und Kommunikationseinrichtung eine Abstraktionsschicht (Geräteabstraktionsschicht) benutzen, die wie eine Schnittstelle (Interface) wirkt und die Kommunikation mit den verschiedensten Messgeräten und Sensoren ermöglicht, falls diese Geräte unterschiedliche Datenformate aufweisen. Die Abstraktionsschicht ermittelt aus dem Datenstrom der angeschlossenen Sensoren die Messwerte und um was für eine Art Sensor/Messgerät (Waage, Blutdruckmesser etc.) es sich handelt.

Entsprechend der Weiterentwicklung von Messgeräten und Sensoren, kann die Abstraktionsschicht an neue Messgeräte und Sensoren bzw. neue Datenformate (Protokolle) angepasst werden, beispielsweise mittels über das Internet heruntergeladener Softwareupdates. Das ermöglicht, dass verschiedenste Messgeräte mit der Datenverarbeitungs- und Kommunikationseinrichtung kommunizieren können, z.B. Blutdruckmessgerät, Waage, Blutzuckermessgerät, Fieberthermometer, Schrittzähler, Körperfettmessgerät, Cholesterinmessgerät, Messgerät für Körperwasser, Bioimpendanzmessgerät, Urinprobenmessgerät, Blutbildmessgerät, Messgeräte zur Analyse von Körperflüssigkeiten und/oder Atem.

Weiter kann die Datenverarbeitungs- und Kommunikationseinrichtung so konfiguriert sein, um eine automatische Identifikation eines Patienten anhand biometrischer Daten durchzuführen. Eine Verknüpfung der von einem Messgerät zur Messung der Vitalparameter erhaltenen Vitalparameter mit biometrischen Daten des Patienten ermöglicht eine eindeutige Identifizierung des Patienten, bzw. Zuordnung zu der Person von der die Vitalparameter aufgenommen wurden. Biometrische Daten können beispielsweise Iris-Scans, Gesichtserkennung, Erkennung der Stimme, oder ein Fingerabdruck sein. Eine Kombination aus einem oder mehreren solcher Parameter ist ebenso möglich und vorteilhaft für eine eindeutige Identifizierung.

Die Datenverarbeitungs- und Kommunikationseinrichtung kann weiterhin mit den entsprechenden Mitteln ausgestattet sein, um diese gerade genannten, biometrischen Parameter aufzunehmen, wie beispielsweise ein Fingerabdruckscanner/sensor, eine Kamera zur Gesichtserkennung oder Mittel zur Stimmerkennung.

Eine weitere Variante der Methode der Identifizierung anhand biometrischer Daten ist, dass über vorhandene Messwerte mittels "künstlicher Intelligenz" eine Datenkurve des Patienten angelernt, also ein "Fingerabdruck" seiner Vitalparameter erstellt wird. Mit Hilfe der gelernten Daten wird dann ein Patient eindeutig identifiziert. Diese Analyse der Messwerte wird auf dem Datenbankserver durchgeführt, da nur hier die entsprechende Rechenleistung zur Verfügung steht. Die Datenverarbeitungs- und Kommunikationseinrichtung erhält aus der, auf dem Server berechneten, Analyse einen Datensatz zurück, der auf der Datenverarbeitungs- und Kommunikationseinrichtung verbleibt und charakteristisch für den jeweiligen Patienten ist. Für jeden Benutzer der Datenverarbeitungs- und Kommunikationseinrichtung wird ein solcher charakteristischer Datensatz abgespeichert. Geht eine Messung in der Datenverarbeitungs- und Kommunikationseinrichtung ein, wird sie mit den charakteristischen Datensätzen verglichen. Wird eine Übereinstimmung gefunden, kann eine eindeutige Zuordnung des Messwertes zu dem Patienten erfolgen.

Sollten keine Mittel zur Erfassung biometrischer Daten vorhanden oder diese defekt sein, wird als "Fallback"-Lösung immer eine manuelle Eingabe zur Identifizierung gewählt.

Die Datenverarbeitungs- und Kommunikationseinrichtung kann Mittel besitzen, die es ermöglichen, Orts- und Zeitangaben aufzunehmen und damit den Patientendaten Informationen über Zeitpunkt der Messung und/oder den Ort der Messung hinzufügen. Geeignete Mittel für eine Ortsbestimmung sind beispielsweise GPS Empfänger oder andere Ortsbestimmungseinrichtungen, die in der Datenverarbeitungs- und Kommunikationseinrichtung integriert sein können. Die ermittelten Orts- und/oder Zeitangaben können einem Datensatz, bestehend aus Vitalparametern des Patienten und Daten, die den Patienten eindeutig den gemessenen Vitalparametern zuordnen, als Metadaten hinzugefügt werden. Vorteilhaft kann dies sein, wenn sich beispielsweise der Patient an Orten mit unterschiedlichen Umweltbedingungen aufhält, wie beispielsweise im Urlaub in einer heißen, kalten oder feuchten Umgebung. Sollten sich evtl. Auffälligkeiten bei den Messwerten ergeben, ist es möglicherweise nützlich zu wissen, wo sich der Patient während der Messung der Vitalparameter aufhält bzw. aufgehalten hat, um ggf. die Therapie an die jeweiligen Bedingungen anzupassen.

Die Datenverarbeitungs- und Kommunikationseinrichtung kann sowohl stationär als auch transportabel ausgelegt werden. Dies ermöglicht es, dass sich mehrere Patienten eine Datenverarbeitungs- und Kommunikationseinrichtung teilen können. Eine transportable Datenverarbeitungs- und Kommunikationseinrichtung kann dabei mit oder über ein gängiges Smartphone, Tablet-PC oder Laptop zusammenwirken bzw. kommunizieren.

Zusätzlich zur Datenverarbeitungs- und Kommunikationseinrichtung wird ein Verfahren offenbart, mit dem die zentrale Überwachung von zumindest einem Vitalparameter zumindest eines Dialysepatienten in einer dialysefreien Zeit möglich ist.

In einem ersten Schritt wird dabei ein Verbindungsaufbau zwischen einem Sensor oder einem Messgerät zur Messung mindestens eines Vitalparameters zu einer Datenverarbeitungs- und Kommunikationseinrichtung durchgeführt. Nachdem die Verbindung hergestellt ist, kann ein Vitalparameter eines Patienten in der therapiefreien/dialysefreien Zeit gemessen werden. Messgrössen können dabei Blutdruck, Gewicht, Blutzucker, Körpertemperatur, körperliche Aktivität, Körperfett, Cholesterinspiegel, Anteil Körperwasser, Bioimpedanz, Urin, Blutbild und/oder Analyse von Körperflüssigkeit und/oder Atem sein. Nach Messung eines oder mehrerer Vitalparameter wird eine Übertragung zur Datenverarbeitungs- und Kommunikationseinrichtung durchgeführt. In der Datenverarbeitungs- und Kommunikationseinrichtung wird, nach erfolgter Übertragung, die Zuordnung einer Patienten-ID zu dem Messwert/Vitalparameter durchgeführt, um eine eindeutige Verknüpfung von Messwert/Vitalparameter mit dem Patienten zu bekommen. In einem weiteren Schritt prüft die Datenverarbeitungs- und Kommunikationseinrichtung selbsttätig, ob ein Verbindungsaufbau zum Internet möglich ist, um eine Ferndatenverbindung zu einer zentralen Patientendatenbank aufzubauen. Wird erkannt, dass ein Verbindungsaufbau möglich ist, baut die Datenverarbeitungs- und Kommunikationseinrichtung selbsttätig eine Ferndatenverbindung zu einer zentralen Patientendatenbank auf. Ist die Verbindung zur zentralen Patientendatenbank aufgebaut, erfolgt eine selbsttätige Übertragung eines Datensatzes aus Patienten-ID und dem zumindest einen gemessenen Vitalparameter von der Datenverarbeitungs-und Kommunikationseinrichtung an die zentrale Patientendatenbank.

In der zentralen Patientendatenbank wird der Datensatz als Patientendatensatz abgespeichert, und kann über eine Kommunikationsschnittstelle von Ärzten, Klinikpersonal und/oder dem entsprechenden Patienten jederzeit eingesehen werden.

In einem zusätzlichen Schritt kann es erforderlich sein, dass sich der Patient einmalig an der Datenverarbeitungs- und Kommunikationseinrichtung anmeldet. Es kann auch schon während der Messung der Vitalparameter eine Identifikation des Dialysepatienten übertragen werden, falls ein Messgerät/Sensor entsprechend konfiguriert ist.

Das Messgerät/Sensor besitzt dann eine Kennung, die zur Identifikation des Patienten benutzt werden kann und zusammen mit dem Messwert zur Datenverarbeitungs- und Kommunikationseinrichtung übertragen wird. Diese Option kann sinnvoll sein, wenn die Datenverarbeitungs- und Kommunikationseinrichtung von einer einzelnen Person verwendet wird. Auch hier wird immer eine manuelle Eingabe zur Legitimation des Patienten als "Fallback"-Lösung vorgesehen, wenn anderweitig keine Möglichkeit zur Identifikation besteht.

In einem alternativen Schritt kann die Zuordnung der Daten zu einem Patienten, mittels Erfassung biometrischer Daten des Patienten und Zuordnen der ID gemäß den erfassten, biometrischen Daten durchgeführt werden. Die Datenverarbeitungs- und Kommunikationseinrichtung kann die nötigen Mittel bereitstellen, um diese Daten aufzunehmen (Fingerabdruckscanner, Kamera zur Gesichtserkennung etc.).

Die Daten können auf der Datenverarbeitungs- und Kommunikationseinrichtung zwischengespeichert und, nach erfolgreicher Übertragung an die zentrale Patientendatenbank, von der Datenverarbeitungs- und Kommunikationseinrichtung gelöscht werden.

Zusätzlich können dem Datensatz des Patienten, der aus Vitalparametern und ID-Informationen besteht, von der Datenverarbeitungs- und Kommunikationseinrichtung Metadaten hinzugefügt werden, wobei die Metadaten Zeit- und/oder Ortsdaten enthalten können. Die Datenverarbeitungs- und Kommunikationseinrichtung enthält dabei die notwendigen Mittel um diese Daten dem Patientendatensatz hinzuzufügen.

In einem weiteren Schritt kann die Datenverarbeitungs- und Kommunikationseinrichtung die Daten des Datensatzes, bestehend aus Patienten-ID und Vitalparametern, verschlüsseln. Alternativ oder zusätzlich kann die Datenverarbeitungs- und Kommunikationseinrichtung eine sichere Verbindung zu einem Datenbankserver (zentrale Patientendatenbank) aufbauen. Beide Maßnahmen sind vorteilhaft im Hinblick darauf, dass die Daten personenbezogen und relevant hinsichtlich des Datenschutzes sind.

Gelingt aus irgendeinem Grund die Übertragung des Datensatzes an die zentrale Patientendatenbank nicht, wird von der Datenverarbeitungs- und Kommunikationseinrichtung automatisch solange ein erneuter Verbindungsaufbau initiiert, bis eine erfolgreiche Übertragung stattgefunden hat.

Entsprechend der auf dem Patientenserver gespeicherten Patientendaten kann mit Hilfe von Datenanalyse-Techniken eine Behandlungsempfehlung an den Arzt gegeben werden. Diese Behandlungsempfehlung kann den Arzt, das Pflegepersonal oder auch den Patienten bei der Dialyse unterstützen. Anhand der erhobenen Daten können viele, die Behandlung unterstützende, Behandlungsempfehlungen an den Arzt oder das Pflegepersonal gegeben werden. Dazu zählen unter Anderem: Empfehlung des Ultrafiltrations Profils auf Grundlage von Blutdruckmessungen von Zuhause; Empfehlung des Ultrafiltrations Volumens auf Grundlage von zugenommenem Körperwasser; Empfehlung eines bestimmten Konzentrates; Empfehlung eines bestimmten Leitfähigkeitsprofils; Empfehlung einer bestimmten Leitfähigkeit/Na-Konzentration; Empfehlung einer bestimmten Diät; Empfehlung eines bestimmten Dialysierflüssigkeitsflusses; Empfehlung eines bestimmten Blutflusses; Empfehlung eines bestimmten Dialysators; Empfehlung eines bestimmten Medikaments; Empfehlung einer bestimmten Heparindosis; Empfehlung einer bestimmten Epo-Dosis; Empfehlung eines bestimmten Phosphatbinders; Empfehlung einer bestimmten Einstellung; und/oder Empfehlung, eine bestimmte Option der Dialysemaschine zu nutzen wie z.B. eine BioLogic RR Behandlung, bei Erkennung häufiger und starker Blutdruckabfälle um diesen entsprechend entgegenzuwirken um hypotensive Episoden während der Dialyse zu vermeiden;.

Diese Empfehlungen können auf Grundlage der während der dialysefreien Zeit erhobenen Daten, bzw. auf Basis aller Daten, die für den jeweiligen Patienten in der Datenbank vorhanden sind, ermittelt bzw. ausgesprochen werden. Das kann auch therapiebezogene Daten mit einschliessen. ausgesprochen werden. Die Datenanalyse wird beim Eingang eines neu gemessenen Vitalparameters eines Patienten gestartet. Je nach Art des Messwerts (Blutdruck, Gewicht, Blutzucker) kann ein Analyseprozess, in Abhängigkeit vom gemessenen Vitalparameter, gestartet werden. Der Analyseprozess ermittelt, auf Basis des gemessenen Vitalparameters, alte Messwerte des gleichen Typs und desselben Patienten. Über bestimmte mathematische Methoden (Mittelwert, Standardabweichung, Varianz, Frequenzinformationen (FFT=Fast Fourier Transformation), wird versucht, besondere Merkmale (Features) dieser Daten zu ermitteln.

Danach wird ein Modell angeglichen, auf den Wert angewendet oder eine Vorhersage und/oder Klassifizierung durchgeführt. Auf Grundlage dieses Verarbeitungsschrittes wird eine Behandlungsempfehlung ermittelt, die in einem nächsten Schritt in der Patientendatenbank gespeichert werden kann. Die abgespeicherten Behandlungsempfehlungen können dem behandelnden Arzt angezeigt werden, wenn er das nächste Mal die Patientendaten abruft oder wenn er die Behandlungsempfehlungen explizit anfordert. Auch dem Patienten oder dem behandelnden Personal kann ermöglicht werden, die Behandlungsempfehlungen einzusehen.

Die Erfindung wird nachstehend anhand bevorzugter Ausführungsbeispiele unter Bezugnahme auf die begleitenden Zeichnungen näher erläutert.
Fig.1 zeigt den Aufbau einer Datenverarbeitungs- und Kommunikationseinrichtung (Datenbox) mit angeschlossenen Geräten;
Fig. 2 zeigt ein Flussdiagramm eines Messablaufs;
Fig. 3 zeigt ein Flussdiagramm einer Patientenanfrage; und
Fig. 4 zeigt ein Flussdiagramm einer Arztanfrage
Fig. 5 zeigt den Ablauf zu einer Behandlungsempfehlung
Fig. 6 zeigt die Analyseeinheit auf dem Server

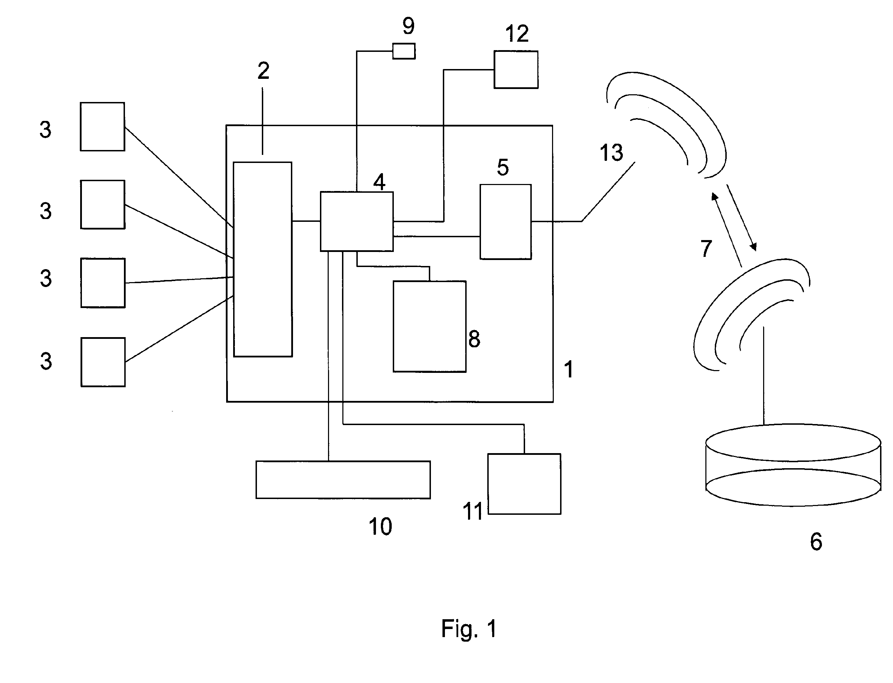

Fig.1 zeigt den Aufbau einer Datenverarbeitungs- und Kommunikationseinrichtung 1 (im Folgenden lediglich Datenbox 1 genannt). Dabei nehmen eines oder mehrere Messgeräte 3 Vitalparameter eines Patienten auf. Es kann sich bei den Messgeräten 3 um verschiedenste Messgeräte handeln, wie beispielsweise eine Waage, ein Blutdruckmessgerät oder andere Messgeräte für Vitalparameter.

Das eine oder auch mehrere der Messgeräte 3 ist mit der Datenbox 1 über eine drahtlose oder drahtgebundene Verbindung gekoppelt. Die Datenbox 1 empfängt die, von den Messgeräten 3 gemessenen und gesendeten, Vitalparameter eines Patienten.

Bevor die Messdaten von Messgerät 3 in der Datenbox 1 weiterverarbeitet werden, sorgt eine Geräteabstraktionsschicht 2 dafür, dass die vom Messgerät 3 eingehenden Messdaten ein Datenformat aufweisen, welches von der Datenbox 1 weiterverarbeitet werden kann. Da das oder die Messgeräte 3 von verschiedenen Herstellern stammen können, ist es nicht immer gegeben, dass das Datenformat, beispielsweise von Gewichtsmessgeräten (Waagen) zweier unterschiedlicher Hersteller, gleich ist. Hierfür enthält die Datenbox 1 eine Geräteabstraktionsschicht 2. Die Geräteabstraktionsschicht 2 ist so aufgebaut, dass sie verschiedene Datenprotokolle von Messgeräten 3 unterschiedlicher Hersteller aufnimmt und in ein Datenformat umsetzt, das von der Datenbox 1 weiterverarbeitet werden kann.

Nach Passieren der Geräteabstraktionsschicht 2 werden in einer Datenverarbeitungseinheit 4 zu den Vitalparametern Identifikationsdaten zugefügt, die es ermöglichen, den Patienten eindeutig seinen gemessenen Vitalparametern zuzuweisen.

Im einfachsten Fall hat sich der Patient über eine Tastatur 10 an der Datenbox 1 angemeldet, beispielsweise mit seinem Namen und/oder einem Passwort, welches nur er kennt. Mit diesen Daten erzeugt die Datenverarbeitungseinheit 4, zusammen mit den Vitalparametern, einen personalisierten Datensatz des Patienten.

Für die automatische Zuordnung der gemessenen Vitalparameter zu einem Patienten besitzt die Datenbox 1 optional weitere Möglichkeiten, um auf biometrischem Wege eine Zuordnung der Vitalparameter zum Patienten durchzuführen. Eine Kamera 9, die (optional) in Kommunikation mit der Datenbox 1 steht, führt eine Gesichtserkennung oder eine Iriserkennung durch. Anhand dieser biometrischen Daten lässt sich eine eindeutige Zuordnung zum Patienten herstellen. Analog erfolgt die Identifikation mittels eines Fingerabdrucks über einen (ebenfalls optionalen) Fingerabdrucksensor 11.

In einer weiteren Variante werden dem Datensatz zusätzlich Metadaten hinzugefügt. Diese Metadaten sind beispielsweise Ortsdaten, Zeitdaten oder andere Informationen die wichtig in Zusammenhang mit den Vitalparametern sind. Die Ortsdaten werden von einem mit der Datenbox 1 verbundenen GPS Empfänger 12 oder einem anderen, dafür geeigneten Gerät, empfangen. Diese Ortsdaten werden zusätzlich in der Datenverarbeitungseinheit 4 dem Datensatz, bestehend aus Vitalparametern und Identifikationsdaten, hinzugefügt.

Der komplette Datensatz wird durch die Datenverarbeitungseinheit 4 verschlüsselt und im Speicher 8 zwischengespeichert.

Die Datenbox 1 baut jetzt mit den Mitteln zur Detektion und Aufbau der Internetverbindung 5 eine Verbindung ins Internet 7 auf um sich mit dem Patientenserver 6 verbinden zu können. Optional wird, wenn es aus Gründen der Verbindungsqualität erforderlich ist, zusätzlich eine mit der Datenbox 1 verbundene Antenne 13 beim Verbindungsaufbau benutzt. Die

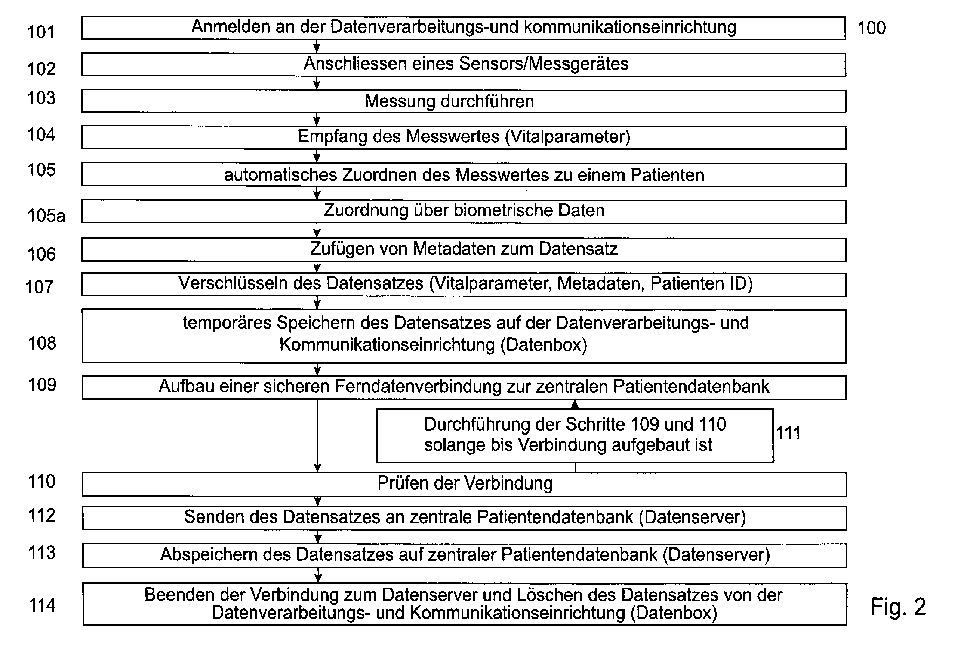

Der Ablauf einer Messung und eines Übertragungsvorgangs ist in Fig. 2 in Form eines Flussdiagramms gezeigt.

Zunächst meldet sich der Patient im Schritt 101 an der Datenbox 1 an. Im Schritt 102 werden ein oder mehrere Messgeräte 3, welche der Patient vorhält, an die Datenbox 1 angeschlossen. Das geschieht kabelgebunden oder kabellos. In Schritt 103 führt der Patient die Messung durch (Gewichtsmessung, Blutdruck etc.). Die Datenbox 1 empfängt in Schritt 104 die Messdaten (Vitalparameter) vom Messgerät 3.

Da sich der Patient in Schritt 101 angemeldet hat, erfolgt automatisch eine Zuordnung des Patienten zu den Messdaten 105. Alternativ, wenn sich mehrere Benutzer eine Datenbox zur Aufnahme ihrer Daten teilen, kann im Schritt 105a eine automatische Identifizierung eines Patienten über biometrische Daten erfolgen. Alternativ, wenn sich mehrere Benutzer eine Datenbox 1 zur Aufnahme von Daten teilen und keine Mittel zur biometrischen Identifikation bereitstehen oder aus irgendeinem Grund nicht einsatzfähig sind, kann eine Identifizierung auch durch manuelle Eingabe erfolgen. Dazu kann dann jeder Benutzer vor einer Messung seiner Parameter seine persönlichen Daten eingeben, beispielsweise über eine an der Datenbox 1 angeschlossene Tastatur und bestätigen, dass die jetzt gemessenen Daten seiner Person zuzuordnen sind. Die Datenbox 1 kann dann so konfiguriert sein, dass die manuelle Identifikation bei nicht vorhandenen oder nicht funktionierenden biometrischen Sensoren generell notwendig ist.

In Schritt 106 werden (optional) Metadaten wie Ortsdaten, Zeitangaben (der Messung) oder andere, für den Arzt wichtige Daten, hinzugefügt. Aus Sicherheitsgründen bzw. Datenschutz wird in Schritt 107 der gesamte Datensatz, bestehend aus gemessenen Vitalparametern, Identifikationsdaten und Metadaten, verschlüsselt und in Schritt 108 in einem Speicherbereich 8 der Datenbox 1 zwischengespeichert.

Die Datenbox 1 startet in Schritt 109 den Aufbau einer Datenverbindung zur zentralen Patientendatenbank und prüft die Verbindung 110. Optional, wenn die Verbindung nicht aufgebaut werden kann, wird in Schritt 111 solange der Ablauf von Schritt 109 und 110 wiederholt, bis eine Verbindung aufgebaut ist. Wenn beispielsweise der Patient in seiner Wohnung aus irgendwelchen Gründen keinen Internetzugang hat oder dieser gestört ist, wird, wenn sich der Patient mit seiner Datenverarbeitung- und Kommunikationseinrichtung an einem geeigneten Ort für den Aufbau einer Verbindung zum Internet befindet, ein neuer Verbindungsversuch gestartet.

Wenn eine Verbindung aufgebaut ist, wird der zwischengespeicherte Datensatz in Schritt 112 an die zentrale Patientendatenbank (Datenserver) 6 geschickt und dort in Schritt 113 abgespeichert. In Schritt 114 wird nach erfolgreicher Übertragung die Verbindung zum Server beendet und der zwischengespeicherte Datensatz von der Datenbox 1 gelöscht.

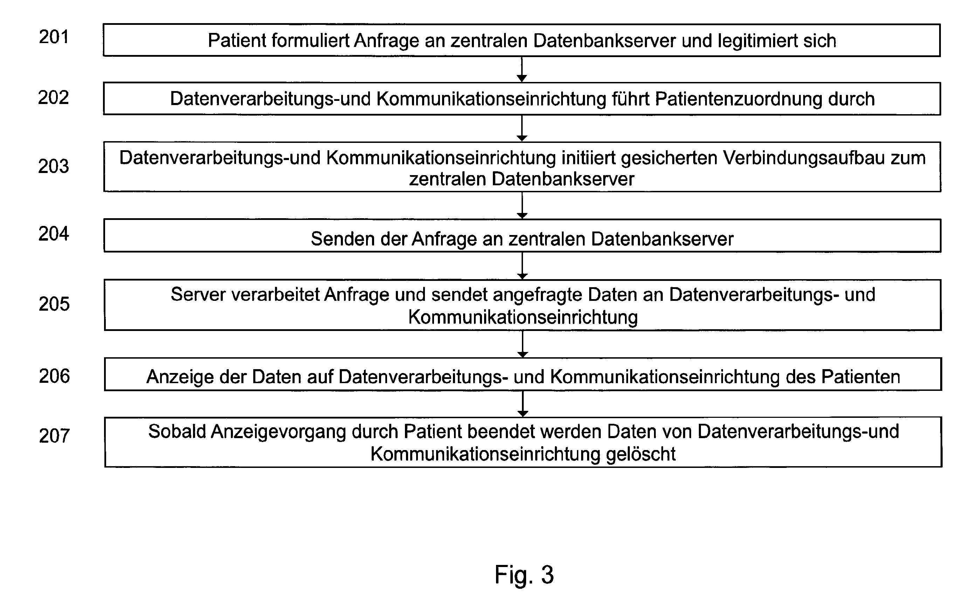

Fig. 3 zeigt den Ablauf einer Patientenanfrage an den zentralen Datenbankserver.

Zum Beispiel möchte sich ein Patient mit Hilfe seiner Datenbox 1 die letzten Aufnahmen seines Blutdrucks anschauen. Dazu formuliert er mit Hilfe entsprechender Eingabemittel in der Datenbox 1 eine Abfrage, wobei er sich auch durch die Eingabe seiner persönlichen Daten gegenüber der zentralen Patientendatenbank 6 legitimiert (Schritt 201). Es folgt, anhand der vom Patienten eingegebenen Legitimationsdaten, eine Patientenzuordnung (Schritt 202). Die Datenbox 1 initiiert jetzt einen Verbindungsaufbau zum Internet (Schritt 203). Ist dies geschehen wird die Anfrage, über eine gesicherte Verbindung an den zentralen Patientendatenbankserver 6 gesendet (Schritt 204). Der zentrale Patientendatenbankserver 6 verarbeitet die Anfrage und sendet über die gesicherte Leitung die Daten an die Datenbox 1, die jetzt dem zentralen Patientendatenbankserver 6 den erfolgreichen Empfang meldet (Schritt 205). Die Verbindung zum zentralen Patientendatenbankserver 6 wird daraufhin beendet. Die Daten werden entsprechend den Möglichkeiten der Datenbox 1 grafisch angezeigt (Schritt 206). Nach erfolgreicher Betrachtung der Daten werden diese wieder gelöscht (Schritt 207).

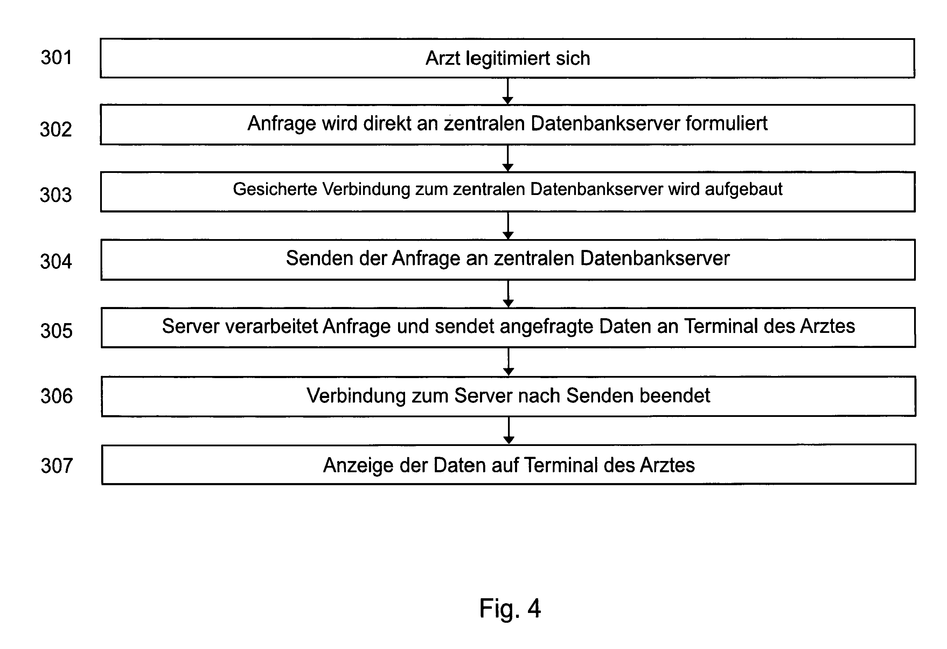

Fig. 4 zeigt einen Ablauf während der Abfrage der Daten durch einen Arzt. Diese Abfrage ist weitestgehend gleich zu der Datenabfrage durch den Patienten. Ein Arzt möchte beispielsweise die Daten eines Patient während der dialysefreien Zeit beobachten. Der Arzt befindet sich im Dialysezentrum und hat von dort aus direkten Zugriff auf den zentralen Patientendatenbankserver 6, beispielsweise über einen Computer an seinem Arbeitsplatz. Nachdem er sich gegenüber dem zentralen Patientendatenbankserver 6 legitimiert hat (Schritt 301), formuliert er mit Hilfe einer Software eine Abfrage an den zentralen Patientendatenbankserver 6 (Schritt 302), z.B. möchte er die Gewichtszunahme des Patienten seit der letzten Behandlung einsehen. Nach erfolgreicher Formulierung der Anfrage und entsprechender Legitimierung wird eine gesicherte Verbindung zu dem Patientendatenbankserver 6 aufgebaut (Schritt 303) und es wird die Anfrage an den zentralen Patientendatenbankserver 6 gesendet (Schritt 304). Der Patientendatenbankserver 6 verarbeitet die Anfrage und sendet die Daten zurück an den Arzt (Schritt 305). Nach erfolgreicher Übertragung der Daten an den Arzt wird die Verbindung beendet (Schritt 306) und dem Arzt wird ein grafischer Verlauf der Daten angezeigt (Schritt 307).

Anstelle der zuvor genannten Internetverbindung zwischen Datenbox 1 und zentrale Patientendatenbank 6 können auch Mobilfunkverbindungen zum Datenaustausch genutzt werden.

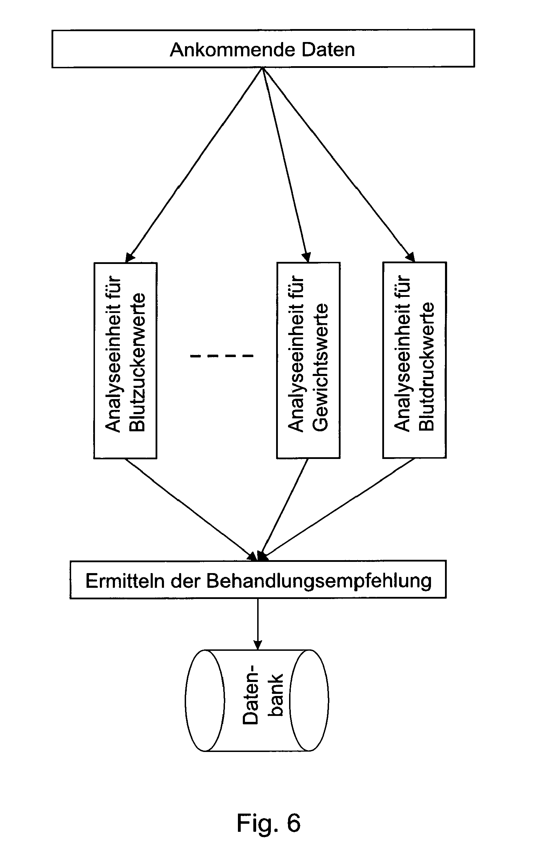

Eine mögliche Analyse-/Empfehlungseinheit auf dem Server ist in Fig. 6 abgebildet. Sie zeigt die Aufteilung der Daten je nach gemessenem Wert. Die Analyse erfolgt auf Grundlage des ermittelten Typs des Messwertes. Die Behandlungsempfehlung kann auch Werte aus anderen Analysen mit einbeziehen, z.B auch Werte, die während einer Therapie erhoben und ebenfalls auf dem Patientenserver/Patientendatenbank 6 abgespeichert wurden. Eine ermittelte Behandlungsempfehlung wird dann in der Patientendatenbank 6 gespeichert, die dem Arzt beim Abrufen der Patientendaten automatisch mit übermittelt wird oder er sie explizit abrufen kann.

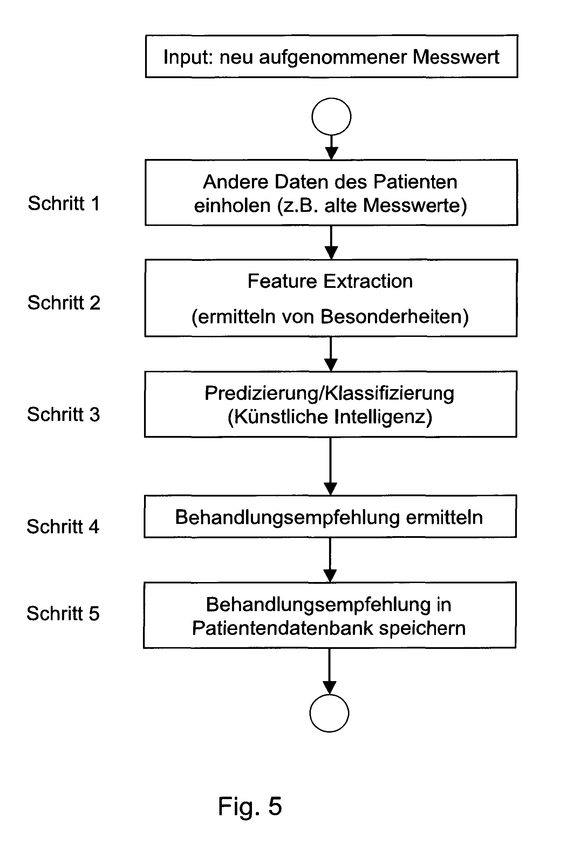

Je nach Art des Messwertes können unterschiedliche Methoden während der Ausführung der Schritte 2 und 3 in Fig. 5 zum Einsatz kommen.

Für Blutdruckmesswerte ist es z.B. interessant, diese im Verlauf zu betrachten und ggf. vorherzusagen, um auf Grundlage des Wertes z.B. ein bestimmtes Ultrafiltrationsprofil während der nächsten Behandlung zu empfehlen.

Bei einem Gewichtsmesswert ist es z.B. interessant, diesen über mehrere Therapien hinweg zu betrachten. Sollte dabei nicht immer die gleiche Reduktion auf ein bestimmtes ermitteltes Gewicht geschehen, kann empfohlen werden, das Ultrafiltrationsvolumen zu erhöhen.

Bei der Messung eines erhöhten Blutzuckerspiegels über den Lauf der Dialysebehandlung kann eine bestimmte Diät empfohlen werden. Wenn sich der Blutzuckerwert wieder auf ein niedriges Niveau normalisiert hat, kann z.B. die Diät wieder abgesetzt werden.

Bei der Messung eines erhöhten oder bei Erkennung eines ansteigenden Trends des Natriumspiegels, kann eine entsprechende Diät in Kombination mit einer bestimmten elektrischen Leitfähigkeit (des Blutes oder der Dialysierflüssigkeit) empfohlen werden.

Daher sind, unter anderem, folgende Analysemethoden interessant:
- Automatische Modellbildung (durch einfache Prozesse aber auch durch Künstliche Intelligenz)
- Ermitteln von Besonderheiten (Features) einer Reihe von Messwerten, wie z.B. Mittelwert, Standardabweichung, Varianz, Frequenzinformationen (FFT)=Fast Fourier Transformation
- Vorhersage von Modellen, Zeitreihen von Messwerten
- Empfehlen von Behandlungen z.B. über Entscheidungsbäume oder Klassifikationsmethoden
- Vergleich der Daten mit anderen Patientendaten, um so eine mögliche Behandlung zu ermitteln.

Durch Anwendung der genannten Analysemethoden auf die Messwerte des Patienten kann demnach eine Behandlungsempfehlung ermittelt werden. Diese ist auf dem Patientenserver abgespeichert und steht dem Arzt oder auch dem Patienten zur Verfügung um entsprechend die Therapie anzupassen.

### Bezugszeichenliste

- 1: Datenbox
- 2: Geräteabstraktionsschicht
- 3: Sensoren/Messgerät
- 4: Datenverarbeitungseinheit
- 5: Mittel zur Detektion und Aufbau der Internetverbindung
- 6: Datenbankserver (zentrale Patientendatenbank)
- 7: Internetverbindung
- 8: Speicherbereich
- 9: Kamera
- 10: Tastatur
- 11: Fingerabdrucksensor
- 12: Ortsdatenempfänger (z.B. GPS)
- 13: Antenne

- 100: Ablauf bei der Datenaufnahme und Speicherung
- 101: Anmelden an der Datenbox 1
- 102: Anschließsen eines Sensors/Messgerätes
- 103: Messung durchführen
- 104: Empfang der Messwertes (Vitalparameter)
- 105: automatisches Zuordnen des Messwertes zu einem Patienten
- 105a: optional: Zuordnung des Messwertes über biometrische Daten
- 106: optional: Zufügen von Metadaten zum Datensatz
- 107: verschlüsseln des Datensatzes (Vitalparameter, Metadaten, Patienten ID)
- 108: temporäres Speichern des Datensatzes auf Datenbox
- 109: Aufbau einer sicheren Ferndatenverbindung zur zentralen Patientendatenbank.
- 110: Prüfen der Verbindung
- 111: optional: Durchführung der Schritte 109 und 110 solange bis Verbindung aufgebaut ist
- 112: Senden des Datensatzes an zentrale Patientendatenbank (Datenserver)
- 113: Abspeichern des Datensatzes auf zentraler Patientendatenbank (Datenserver)
- 114: Beenden der Verbindung zum Datenserver und Löschen des Datensatzes von der Datenbox

- 201: Anfrageformulierung und Legitimation
- 202: Durchführung der Patientenzuordnung
- 203: gesicherter Verbindungsaufbau zum Server
- 204: Anfrage an Server senden
- 205: Verarbeiten der Anfrage und Senden der Daten
- 206: Daten beim Patienten anzeigen
- 207: Beenden des Anzeigevorgangs und löschen der Daten beim Patient

- 301: Arzt legitimiert sich
- 302: Direkte Anfrage an Datenbankserver wird formuliert
- 303: Aufbau einer sicheren Verbindung zum Server
- 304: Senden der Anfrage an Server
- 305: Verarbeiten der Anfrage und Daten an Arzt senden
- 306: Verbindung zum Server beenden
- 307: Anzeige der Daten auf Terminal des Arztes

## Patentansprüche

1. Patientendatenüberwachungssystem zum zentralen Überwachen von zumindest einem Vitalparameter zumindest eines Dialysepatienten in der therapiefreien Zeit, das aufweist:
eine zentrale Patientendatenbank (6) zum Abspeichern von Patientendaten, welche über eine Kommunikationsschnittstelle von Ärzten, Klinikpersonal und/oder dem entsprechenden Patienten jederzeit einsehbar sind; und
zumindest ein, von einem Patienten in der therapiefreien Zeit mitgeführtes oder zu Hause vorgehaltenes, Messgerät (3) zum Messen zumindest eines Vitalparameters des zumindest einen Patienten,
**gekennzeichnet durch**:
eine von dem zumindest einen Patienten in der dialysefreien Zeit mitgeführte oder zu Hause vorgehaltene, insbesondere transportable, Datenverarbeitungs- und Kommunikationseinrichtung (1), welche jedem gemessenen und über eine Datenverbindung vom dem zumindest einen Messgerät (3) erhaltenen Vitalparameter eine entsprechende Patienten-ID zuweist, selbsttätig eine Ferndatenverbindung zu der zentralen Patientendatenbank (6) aufbaut und selbsttätig, sobald die Verbindung zur zentralen Patientendatenbank (6) aufgebaut ist, einen Datensatz aus Patienten-ID und dem zumindest einen gemessenen Vitalparameter an die zentrale Patientendatenbank (6) überträgt.

2. Patientendatenüberwachungssystem nach Anspruch 1, wobei die Datenverarbeitungs- und Kommunikationseinrichtung (1) Mittel aufweist, die eine Unterscheidung von mehreren Patienten, die die Datenbox 1 zur zentralen Überwachung von Vitalparametern benutzen, ermöglicht und jedem einzelnen Patienten eine eindeutige Patienten-ID zuordnet.

3. Patientendatenüberwachungssystem nach Anspruch 1, wobei die Datenverarbeitungs- und Kommunikationseinrichtung (1) so konfiguriert ist, dass sie eine Kommunikation mit einer Vielzahl von Messgeräten (3) und Sensoren ermöglicht, wobei die Messgeräte (3) und Sensoren beliebige Datenprotokolle aufweisen können.

4. Patientendatenüberwachungssystem nach einem der Ansprüche 1 bis 3, wobei die Datenverarbeitungs- und Kommunikationseinrichtung (1) Mittel aufweist, die eine Identifikation eines Patienten anhand biometrischer Daten ermöglicht, insbesondere eine Kamera und/oder ein Fingerabdrucksensor und/oder eine Stimmerkennungseinrichtung aufweist.

5. Patientendatenüberwachungssystem nach einem der Ansprüche 1 bis 4, wobei die Datenverarbeitungs- und Kommunikationseinrichtung (1) Mittel besitzt, die es ermöglichen, dem Patientendatensatz, bestehend aus Vitalparametern und Identifizierungsdaten, Informationen über Zeitpunkt der Messung und/oder den Ort der Messung hinzuzufügen (4).

6. Patientendatenüberwachungssystem nach einem der Ansprüche 1 bis 5, wobei die Patientendatenbank (6) so konfiguriert ist, um eine Analyse und/oder Bewertung der auf der Patientendatenbank (6) gespeicherten und zu einem Patienten gehörigen Patientendaten auf der Basis eines von demselben Patienten neu empfangenen Vitalparameters durchzuführen.

7. Patientendatenüberwachungssystem nach Anspruch 6, wobei die Patientendatenbank (6) so konfiguriert ist, um das Ergebnis der analysierten und/oder bewerteten Patientendaten auf der Patientendatenbank (6) so zu speichern, dass das Ergebnis dem entsprechenden Patienten zugeordnet ist.

8. Verfahren (100) zum zentralen Überwachen von zumindest einem Vitalparameter zumindest eines Dialysepatienten in der dialysefreien Zeit, das die Schritte aufweist:
a) Aufbauen einer Verbindung zwischen mindestens einem Sensor oder Messgerät (3) zur Messung mindestens eines Vitalparameters mit einer Datenverarbeitungs- und Kommunikationseinrichtung (1) (S102);
b) Messen zumindest eines Vitalparameters des Patienten durch den mindestens einen Sensor oder Messgerät (3) in der dialysefreien Zeit (S103);
c) Übertragen des Messwertes zu der Datenverarbeitungs- und Kommunikationseinrichtung (1) (S104);
d) Zuordnen einer dem Patienten entsprechenden Patienten-ID zu jedem gemessenen Vitalparameter auf der Datenverarbeitungs- und Kommunikationseinrichtung (1) (S105);
e) Prüfen und selbsttätiges Aufbauen einer Ferndatenverbindung zu einer zentralen Patientendatenbank (6) durch die Datenverarbeitungs- und Kommunikationseinrichtung (1) (S109);
f) Selbsttätiges Übertragen eines Datensatzes aus Patienten-ID und dem zumindest einen gemessenen Vitalparameter von der Datenverarbeitungs- und Kommunikationseinrichtung (1) an die zentrale Patientendatenbank (6), sobald die Verbindung zur zentralen Patientendatenbank (6) aufgebaut ist (S112); und
g) Abspeichern des Datensatzes als Patientendaten in der zentralen Patientendatenbank (6), so dass dieser über eine Kommunikationsschnittstelle von Ärzten, Klinikpersonal und/oder dem entsprechenden Patienten jederzeit einsehbar ist.

9. Verfahren nach Anspruch 8, **dadurch gekennzeichnet, dass** das Verfahren zusätzlich den Schritt enthält, dass sich der Patient an der Datenverarbeitungs- und Kommunikationseinrichtung (1) anmeldet (S101).

10. Verfahren nach Anspruch 8 oder 9, **dadurch gekennzeichnet, dass** der Verfahrensschritt d) durch den mindestens einen Sensor oder Messgerät zur Messung des mindestens einen Vitalparameters erfolgt.

11. Verfahren nach einem der Ansprüche 8 bis 10, **dadurch gekennzeichnet, dass** der Verfahrensschritt d) mittels Erfassen biometrischer Daten des Patienten und Zuordnen der ID gemäß den erfassten, biometrischen Daten durchgeführt wird.

12. Verfahren nach einem der Ansprüche 8 bis 11, **dadurch gekennzeichnet, dass** das Verfahren zusätzlich den Schritt enthält, dass die Daten auf der Datenverarbeitungs- und Kommunikationseinrichtung (1) zwischengespeichert werden (108) und nach erfolgreicher Übertragung an die zentrale Patientendatenbank (6) von der Datenverarbeitungs- und Kommunikationseinrichtung (1) gelöscht werden (S113).

13. Verfahren nach einem der Ansprüche 8 bis 12, **dadurch gekennzeichnet, dass** der Verfahrensschritt d) zusätzlich den Schritt enthält, dass dem Datensatz des Patienten von der Datenverarbeitungs- und Kommunikationseinrichtung (1) Metadaten hinzugefügt werden, wobei die Metadaten unter Anderem Zeit- und/oder Ortsdaten enthalten (S106).

14. Verfahren nach einem der Ansprüche 8 bis 13, **dadurch gekennzeichnet, dass** das Verfahren zusätzlich den Schritt enthält, die Daten des Datensatzes aus Patienten-ID und Vitalparametern zu verschlüsseln (S107) und/oder im Verfahrensschritt e) die Verbindung zu einem sicheren Datenbankserver aufgebaut wird (S109).

15. Verfahren nach einem der Ansprüche 8 bis 14, **dadurch gekennzeichnet, dass** im Falle des Fehlschlagens des Verfahrensschrittes e) automatisch solange ein erneuter Verbindungsaufbau versucht wird, bis eine erfolgreiche Übertragung stattgefunden hat (S111).

16. Verfahren nach einem der Ansprüche 8 bis 15, **dadurch gekennzeichnet, dass** das Verfahren zusätzlich den Schritt enthält, dass entsprechend der auf dem Patientenserver (6) gespeicherten Patientendaten, insbesondere automatisch, eine Behandlungsempfehlung gegeben wird und wobei die sich gespeicherten Patientendaten aus während der dialysefreien Zeit gesammelten Daten und/oder aus den während der Therapie gesammelten und auf dem Patientenserver (6) gespeicherten Daten bestehen.
